(19) Europäisches Patentamt
European Patent Office
Office européen des brevets

(11) **EP 3 691 514 B1**

(12) **EUROPEAN PATENT SPECIFICATION**

(45) Date of publication and mention
of the grant of the patent:
**03.12.2025 Bulletin 2025/49**

(21) Application number: **18800040.0**

(22) Date of filing: **02.10.2018**

(51) International Patent Classification (IPC):
***A61B 3/00*** *(2006.01)*

(52) Cooperative Patent Classification (CPC):
**A61B 3/0091; A61B 3/0025**

(86) International application number:
**PCT/EP2018/076717**

(87) International publication number:
**WO 2019/068671 (11.04.2019 Gazette 2019/15)**

(54) **METHOD AND SYSTEM FOR ADAPTING THE VISUAL AND/OR VISUAL-MOTOR BEHAVIOUR OF A PERSON**

VERFAHREN UND SYSTEM ZUM ANPASSEN DES VISUELLEN UND/ODER
VISUELL-MOTORISCHEN VERHALTENS EINER PERSON

PROCÉDÉ ET SYSTÈME PERMETTANT D'ADAPTER LE COMPORTEMENT VISUEL ET/OU
VISUEL-MOTEUR D'UNE PERSONNE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **02.10.2017 EP 17306314**

(43) Date of publication of application:
**12.08.2020 Bulletin 2020/33**

(73) Proprietors:
- **Essilor International**
**94220 Charenton-le-Pont (FR)**
- **Université de Montréal**
**Montreal, QC H3T 1J4 (CA)**

(72) Inventors:
- **DROBE, Björn**
**94220 Charenton-Le-Pont (FR)**
- **FAUBERT, Jocelyn**
**Montréal**
**Québec H3T 1J4 (CA)**
- **GIRAUDET, Guillaume**
**94220 Charenton-Le-Pont (FR)**

(74) Representative: **Cabinet Novitech**
**188 Grande rue Charles de Gaulle**
**94130 Nogent-sur-Marne (FR)**

(56) References cited:
**WO-A1-2015/124574     WO-A1-2017/144933
JP-A- 2003 075 783**

**Description**

FIELD OF THE INVENTION

The invention relates to a method and a system for adapting the visual and/or visual-motor behaviour of a person. The invention also relates to a computer program product comprising one or more stored sequences of instructions that is accessible to a processor and which, when executed by the processor, causes the processor to carry out the steps of the methods according to the invention.

BACKGROUND OF THE INVENTION

Ophthalmic lenses intended to be held in a frame usually involve a prescription. The ophthalmic prescription can include a positive or negative power prescription as well as an astigmatism prescription. These prescriptions correspond to corrections enabling the wearer of the lenses to correct defects of his vision. A lens is fitted in the frame in accordance with the prescription and with the position of the wearer's eyes relative to the frame. WO 2015/124574 A1, WO 2017/144933 A1, JP 2003 075783 A disclose prior art.

WO 2015/124574 A1 discloses a method for optimizing an optical lens device using eye tracking technology to monitor wearer parameters for improved lens design. WO 2017/144933 A1 discloses a method for determining optical parameters of optical devices using portable electronic devices with image acquisition modules. JP 2003-075783 discloses a mechanical attachment mechanism for mounting visual enhancement attachments to eyeglass frames. US 2017/0017083 A1 discloses dynamic lens correction using variable focus elements for AR/VR applications.

For presbyopic wearers, the value of the power correction is different for far vision and near vision, due to the difficulties of accommodation in near vision.

The prescription thus comprises a far-vision power value and an addition representing the power increment between far vision and near vision; this comes down to a far-vision power prescription and a near-vision power prescription. Lenses suitable for presbyopic wearers are often progressive addition lenses.

Progressive addition ophthalmic lenses include a far-vision zone, a near-vision zone and an intermediate-vision zone, a principal progression meridian crossing these three zones. They are generally determined by optimization, based on a certain number of constraints imposed on the different features of the lens.

Nevertheless, progressive addition lenses (PAL) generate aberrations, notably leading to blur, that in particular reduce the field of view and distortion that creates for example the well known swim effect. The design of the lens is necessarily a compromise between these aberrations.

Consequently, some wearers may have difficulties to adapt to the use of progressive addition lenses despite normal binocular vision and other normal clinical findings.

There is a need for simple and reliable method to help the person to accept or not the use of progressive addition lenses.

An aim of the present invention is to propose such solution.

SUMMARY OF THE INVENTION

To this end, the invention proposes a method which is defined in claim 1.

Advantageously, the method according to the invention allows determining a suitable visual and/or visual-motor training to be provided to the person in order to help the person to accept the use of the optical system more quickly and / or more easily. Thus, the method according to the invention helps the person to accept the use of progressive addition lenses and allows reducing the time that could be required for the wearer, ie the person intended to wear the lens to become accustomed to this lens.

The method according to the invention allows to guarantee for each person a rapid and complete adaptation to the wearing of ophthalmic lenses and progressive power lenses in particular.

According to further embodiments that can be considered alone or combined according to all the possible combinations:

- the optical system comprises at least an ophthalmic lens, preferably a progressive addition lens;
- the optical parameter of the ophthalmic lens comprises at least lens design data indicative of a lens design adapted to the person, the lens design comprising at least the dioptric lens design and/or geometrical parameters of the ophthalmic lens and/or prescription data and/or ophthalmic parameters relating to the ophthalmic requirements of the person;
- the person is equipped with the optical system during the visual and/or visual-motor behaviour modifying step;
- the method further comprises a person visual parameter measurement step during which the person visual parameter of the person relative to at least one visual and/or visual-motor behaviour of the person is measured;
- the repeating of the modifying and assessing steps in the determining step is based on the measurement(s) of the person visual parameter of the person relative to at least one visual and/or visual-motor behaviour of the person;
- the measured value of the person visual parameter is compared to a scale to define a person visual index;
- the person visual parameter is measured upon a perception test carried out on the person;
- the method further comprises a person visual parameter flexibility measuring step, during which the flexibility of the person visual parameter is measured based on the measurement of the person visual parameter;

- the visual and/or visual-motor behaviour modifying step comprises at least:

  • a visual pattern presentation step, during which a set of visual patterns is presented to the person, the set of visual patterns having a visual pattern parameter whose value for each visual pattern varies in the set, the visual pattern parameter being related to the person visual parameter;

  • a reference value providing step, during which a reference value of the visual parameter of the person based on said visual patterns and on said optical parameter of the optical system is provided,

  • a comparing step, during which the person visual parameter and the reference value are compared so as to deduce whether the use of the optical system by the person is adapted when the person is equipped with the optical system and when the visual patterns are looked by the person, and

  • a warning step, during which a warning is provided to the person based on the result of the comparing step;

- the method further comprises a visual and/or visual-motor behaviour adapting efficiency evaluating step, during which the efficiency of the visual and/or visual-motor behaviour adapting is evaluated;

- the method further comprises an optical system information generating step, during which an information related to an optical system adapted to the person is generated based on the efficiency of the visual and/or visual-motor behaviour adapting. Another object of the invention relates to a system for adapting the visual and/or visual-motor behaviour of a person, the system being defined in claim 14.

According to one embodiment of the system, the visual and/or visual-motor behaviour adapting means comprise at least a mobile device, for example a smartphone and the second receiving means comprises a camera embedded in the mobile device configured to receive the person visual parameter.

The invention also relates to a computer program product comprising one or more stored sequences of instructions that are accessible to a processor and which, when executed by the processor, causes the processor to carry out the steps of the method according to the invention.

The invention also relates to a computer-readable storage medium having a program recorded thereon; where the program makes the computer execute the method of the invention.

The invention further relates to a device comprising a processor adapted to store one or more sequence of instructions and to carry out at least one of the steps of the method according to the invention.

Unless specifically stated otherwise, as apparent from the following discussions, it is appreciated that throughout the specification discussions utilizing terms such as "computing", "calculating", or the like, refer to the action and/or processes of a computer or computing system, or similar electronic computing device, that manipulate and/or transform data represented as physical, such as electronic, quantities within the computing system's registers and/or memories into other data similarly represented as physical quantities within the computing system's memories, registers or other such information storage, transmission or display devices.

Embodiments of the present invention may include apparatuses for performing the operations herein. This apparatus may be specially constructed for the desired purposes, or it may comprise a general purpose computer or Digital Signal Processor ("DSP") selectively activated or reconfigured by a computer program stored in the computer. Such a computer program may be stored in a computer readable storage medium, such as, but is not limited to, any type of disk including floppy disks, optical disks, CD-ROMs, magnetic-optical disks, read-only memories (ROMs), random access memories (RAMs) electrically programmable read-only memories (EPROMs), electrically erasable and programmable read only memories (EEPROMs), magnetic or optical cards, or any other type of media suitable for storing electronic instructions, and capable of being coupled to a computer system bus.

The processes and displays presented herein are not inherently related to any particular computer or other apparatus. Various general purpose systems may be used with programs in accordance with the teachings herein, or it may prove convenient to construct a more specialized apparatus to perform the desired method.

The desired structure for a variety of these systems will appear from the description below. In addition, embodiments of the present invention are not described with reference to any particular programming language. It will be appreciated that a variety of programming languages may be used to implement the teachings of the inventions as described herein.

BRIEF DESCRIPTION OF THE DRAWINGS

Embodiments of the invention will now be described, by way of example only, and with reference to the following drawings in which:

- Figure 1 is an illustration of a chart-flow of a method for adapting the visual and/or visual-motor behaviour of a person according to the invention;

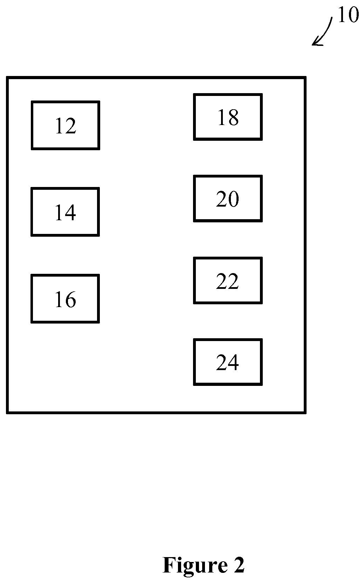

- Figure 2 is a schematic representation of a device configured to implement the method according to an embodiment of the invention.

Elements in the figures are illustrated for simplicity and clarity and have not necessarily been drawn to scale. For example, the dimensions of some of the elements in the figure may be exaggerated relative to other elements to help improve the understanding of the embodiments of the present invention.

DETAILED DESCRIPTION OF PREFERRED EMBODIMENTS

An object of the invention relates to a method for adapting the visual and/or visual-motor behaviour of a person. In the sense of the invention, adapting the visual and/or visual-motor behaviour of a person means determining and providing a suitable visual and/or visual-motor training to the person to help the person to accept the use of the optical system more quickly and / or more easily.

As well-known, the visual behaviour of a person refers to any behavioural reaction/decision or feeling of the person to visual signals or stimuli, as indicated for example in the article of S. Gong et al. published in Image and Vision Computing 20(2002) 873-888.

Furthermore, the visual-motor behaviour of a person relates to any visual behaviour that guides or induces a motor action/response/decision, for examples a pointing task, a walking task... For example, the article of O. Eloka et al. published in Vision Research 51(2011) 925-931 relates on the visual guidance of grasping in response to the object shape.

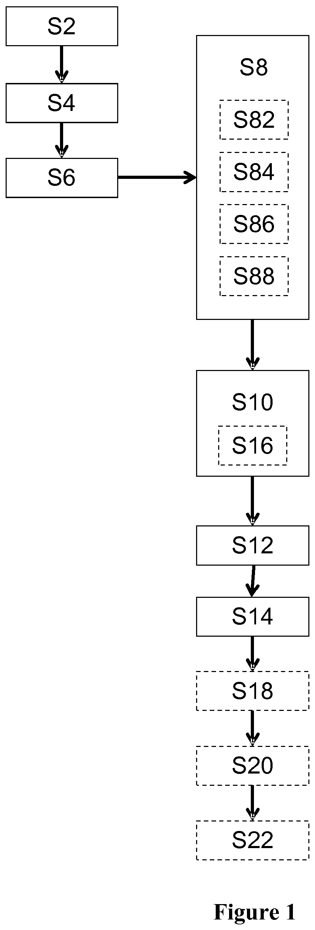

With reference to figure 1, the method for adapting the visual and/or visual-motor behaviour of a person according to the invention comprises at least:

- an optical parameter providing step S2,
- a person visual parameter providing step S4,
- a target value providing step S6,
- a visual and/or visual motor behaviour modifying step S8,
- a person visual parameter assessing step S10,
- a determining step S12, and
- an adapting step S14.

During the optical parameter providing step S2, an optical parameter characterizing an optical system is provided. The optical system is intended to equip the person.

According to an embodiment the optical system comprises at least an ophthalmic lens, and preferably a progressive addition lens. In this case, the optical parameter of the ophthalmic lens may comprise at least lens design data indicative of a lens design adapted to the person, the lens design comprising at least the dioptric lens design and/or geometrical parameters of the ophthalmic lens and/or prescription data and/or ophthalmic parameters relating to the ophthalmic requirements of the person.

During the person visual parameter providing step S4, a person visual parameter indicative of a visual parameter of the person relative to a given visual and/or visual-motor behaviour of the person is provided.

Preferably, the person visual parameter comprises:

- a visual behaviour parameter indicative of the visual behaviour of the person; and/or
- a visual-motor parameter indicative of the visual-motor behaviour of the person, preferably visual-motor coordination data representative of the visual-motor coordination of the person; and/or
- a visual sensitivity parameter, indicative of the visual sensitivity of the person, preferably optical distortion sensitivity data representative of the sensitivity of the person to optical distortions and/or blur tolerance data representative of the tolerance of the person to blur; and/or
- a visual performance parameter indicative of the visual performance of the person; and/or
- a curvature visual perception data representative of the visual perception of the person for curvature shapes.

The person visual parameter providing step S4 advantageously comprises a reference person visual parameter assessing step, during which the person visual parameter is assessed for a reference visual and/or visual-motor behaviour of the person.

Preferably, the reference person visual parameter is assessed by measurement. For example, the measured value of the reference person visual parameter is compared to a scale to define a person visual index.

According to another example compatible with the previous one, the reference person visual parameter is measured upon a perception test carried out on the person.

Then, a target value for the person visual parameter related to the optical parameter is provided during the target value providing step S6.

During the visual and/or visual motor behaviour modifying step S8, a reference visual and/or visual-motor behaviour of the person is modified so as to define a modified visual and/or visual-motor behaviour of the person.

The reference visual and/or visual-motor behaviour of the person is modified by providing a visual and/or visual-motor training to the person. Advantageously, the training aims to minimize the difference between the reference person visual parameter indicative of the reference visual and/or visual-motor behaviour of the person and the target value.

According to an embodiment, the person may be equipped with the optical system during the visual and/or visual-motor behaviour modifying step S8.

Then, the person visual parameter is assessed for the modified visual and/or visual-motor behaviour of the person during the person visual parameter assessing step S10.

Preferably, the person visual parameter is provided by measurement during a person visual parameter measurement step S16 of the method.

For example, the measured value of the person visual parameter is compared to a scale to define a person visual index.

According to another example compatible with the previous one, the person visual parameter is measured upon a perception test carried out on the person.

During the determining step S12, a suitable visual and/or visual-motor behaviour for the person equipped with the optical system is determined by repeating the modifying and assessing steps so as to minimize the difference between the assessed person visual parameters and the target value.

More particularly, by repeating the modifying and assessing steps, a suitable visual and/or visual-motor training to be provided to the person is determined. Such suitable visual and/or visual-motor training aims to minimize the difference between the reference person visual parameter indicative of the reference visual and/or visual-motor behaviour of the person and the target value in order to help the person to accept the use of the optical system more quickly and / or more easily. Such suitable visual and/or visual-motor training is thus personalize to the optical system and to the person, more particularly to the reference visual and/or visual-motor behaviour of the person in evaluating the person's ability to modify his or her visual and/or visual-motor behaviour.

Then, the visual and/or visual-motor behaviour of the person equipped with the optical system is adapted based on the suitable visual and/or visual-motor behaviour during the adapting step S14, by providing to the person the suitable visual and/or visual-motor training as determined allowing to guarantee for the person a rapid and complete adaptation to the wearing of the optical system, such as ophthalmic lenses and progressive power lenses in particular.

Advantageously, the method can further comprise a person visual parameter flexibility measuring step S18, during which the flexibility of the person visual parameter is measured based on the measurement of the person visual parameter.

Furthermore, the method can further comprise a visual and/or visual-motor behaviour adapting efficiency evaluating step S20. The efficiency of the visual and/or visual-motor behaviour adapting is evaluated during such a visual and/or visual-motor behaviour adapting efficiency evaluating step S20.

Advantageously, the method can also comprise a step S22 for generating an optical system information. During this step S22, an information related to an optical system adapted to the person is generated based on the efficiency of the visual and/or visual-motor behaviour adapting.

According to an embodiment, the visual and/or visual-motor behaviour modifying step S8 comprises at least:

- a visual pattern presentation step S82,
- a reference value providing step S84,
- a comparing step S86, and
- a warning step S88.

During the visual pattern presentation step S82, a set of visual patterns is presented to the person. The set of visual patterns has a visual pattern parameter whose value for each visual pattern varies in the set. The visual pattern parameter is related to the person visual parameter.

Then, a reference value of the visual parameter of the person based on said visual patterns and on said optical parameter of the optical system is provided during the reference value providing step S84.

During the comparing step S86, the person visual parameter and the reference value are compared so as to deduce whether the use of the optical system by the person is adapted when the person is equipped with the optical system and when the visual patterns are looked by the person.

Then, a warning is provided to the person based on the result of the comparing step during the warning step S88.

A system 10 for execution of the method for adapting the visual and/or visual-motor behaviour of a person according to the invention will now be described.

The system 10 comprises first, second and third receiving means 12, 14, 16.

The first receiving means 12 are adapted to receive an optical parameter of an optical system, the optical system being intended to equip the person.

The second receiving means 14 are adapted to receive a person visual parameter indicative of a visual parameter of the person relative to a given visual and/or visual-motor behaviour of the person.

The third receiving means 16 are adapted to receive a target value for the person visual parameter related to the optical parameter.

The system further comprises visual and/or visual-motor behaviour modifying means 18 adapted to modify a reference visual and/or visual-motor behaviour of the person so as to define a modified visual and/or visual-motor behaviour of the person.

Moreover, the system comprises visual parameter assessing means 20 adapted to assess the person visual parameter for the modified visual and/or visual-motor behaviour of the person.

Furthermore, the system comprises determining means 22 adapted to determine a suitable visual and/or visual-motor behaviour for the person equipped with the optical system among the modified visual and/or visual-motor behaviours. The visual and/or visual-motor behaviour modifying means and the visual parameter assessing means are configured to repeat the modification of the reference visual and/or visual-motor behaviour of the person and the assessment of his/her corresponding person visual parameter so as to minimize the difference between the assessed person visual parameters and the target value.

The system comprises adapting means 24 configured to adapt the visual and/or visual-motor behaviour of the person equipped with the optical system based on the suitable visual and/or visual-motor behaviour.

Advantageously, the visual and/or visual-motor behaviour adapting means 24 comprise at least a mobile device, for example a smartphone and the second receiving means comprise a camera embedded in the mobile device configured to receive the person visual parameter.

Examples of implementation of the method according to the invention will now be described.

**Example 1: Blur tolerance**

Reference person visual parameter assessment:

This reference assessment aims to provide information on the reference visual and/or visual-motor behaviour of the person and to make it possible to judge the effectiveness of the adaptation by training.

1. Blur tolerance: a first measurement is made to inform the reference level of blur tolerance of the person.
2. Frequency preference: visual patterns, also called hybrid images, are calculated taking into account the person-screen distance. In the sense of the invention, hybrid images have both low and high spatial frequencies, but the low and high spatial frequencies come from different scenes/images, the high SF of one image/scene being combined with the low SF of another scene/image.

A set of 2N images with N hybrid images and N non-hybrid images is presented to the person. For example, N = 36 and the non-hybrid images comprise 12 images having low spatial frequency (LSF), 12 images having high spatial frequency (HSF) and 12 unfiltered images. A measure of the individual frequency preference, hereinafter referred to as IFP, is established and corresponds to the reference value of the visual and/or visual-motor behaviour parameter of the person. This measurement is given by the following formula:

$$IFP = \frac{T_{H/L}}{Th_{H/L}} \times \frac{1}{1 + \frac{T_{H/L}}{Th_{H/L}}}$$

Wherein:

- $T_{H/L}$ is the ratio of success rates for HSF / LSF images;
- $Th_{H/L}$ is the ratio of success rates for hybrid HSF / LSF images;

Thus, only the preference for the high or low spatial frequencies of the hybrid images are obtained independently of an reference asymmetry between these two spatial frequencies ranges.

Like an eye-head coefficient, a value of IFP is obtained comprised between 0 and 1, 0 and 1 characterizing a total preference for LSF and HSF, respectively. For example, a value of 0.5 means that the person does not have a stronger preference for either of these two spatial frequency ranges.

Training

A second measurement is then carried out taking into account this reference IFP measurement. This second measure aims to place the person in a modified visual and/or visual motor behaviour corresponding to the target value of the person visual parameter.

Thus, if it is desired to make the person more tolerant to blurring, his preference must be directed towards low spatial frequencies. Of course, it will be the inverse approach in the case of myopic children whose blur tolerance is to be reduced in order to improve the accommodative control.

This will then be done by exacerbating the preference for the HSF and then causing the system to re-use the LSF preferentially and progressively.

In the case of a visual and/or visual-motor behaviour of a person having a reference preference of 0.4, that is to say a slight tendency to prefer the LSF of the hybrid images, should be trained to make the person more sensitive to the HSF of the hybrid images and then, by training, redirect the person to a preference for the BSF of the hybrid images. For example increasing the contrast of the images, decreasing the illumination of the room in which the measurement is carried out, and/or offseting the cut-off frequency of the highpass filtering to the HSF... move the preference towards HSF.

The second measurement therefore specifies the new IFP, shifted towards the high spatial frequencies. The objective of the training sessions (the visual and/or visual-motor behaviour modified steps) will then be to reduce this preference towards equality or even towards LSF.

In this case, hybrid images are generated and comprise noise in high spatial frequencies and a natural scene for LSF. During the training sessions, the number and type of scenes of the hybrid images can change from one training session to another.

The training consists in exposing the person to hybrid images, some of which are noisy hybrid images as specified above. The proportion of noisy images changes during the sessions according to the level of performance obtained according to the following law:

$$NP(\%) = 10 + \left( \frac{IFP_{max}}{IFP_p} \times 90 \right)$$

wherein:

- NP is the new proportion,
- $IFP_P$ is the IFP with the proportion of noisy hybrid images of the previous session and
- $IFP_{max}$ is the IFP considered to be maximal which is between 0 and 0.1.

Consequently, at the end of the training sessions, in an ideal evolution the subject will have performances so good in the LSF that only hybrid images with noisy HSF will be proposed in the last session.

Final person visual parameter assessment:

This final assessment aims to inform about the visual and/or visual-motor behaviour of the person after training and to allow to evaluate the effectiveness of the training.

1. Blur tolerance: the last measurement is carried out, as the first one, in order to inform about the level of blur tolerance at the end of training and thus check the result obtained and its suitability with the requirements of the progressive lens envisaged for this person.

2. Frequency preference: a control measure is carried out at the end of the training. Using the images of the very first measure, the person must present this time a very wide preference, or even a total preference for the LSF.

In order to implement this method according to this example of the invention, a suitable device can comprise:

- a screen in front of which the person is positioned,
- means for measuring the distance between the person and the screen, for example a telemeter,
- visual parameter assessing means, for example a human-machine interface or speech recognition software.

**Example 2: Eye-head coordination**

Reference person visual parameter assessment:

This reference assessment aims to provide information on the reference visual and/or visual-motor behaviour of the person and to make it possible to judge the effectiveness of the adaptation by training. For example, the reference person visual parameter assessed is the eye-head coefficient (COT). Indeed, it is known that individuals have different propensity to move either their eyes or their head when successively looking in different directions. Such propensity can be of importance when producing ophthalmic lenses. For example, as disclosed in US 2010/0002191, such propensity can be taken into account in order to determine a compromise between the correction of the foveal vision and that of the peripheral vision when producing an ophthalmic lens. The eye-head coefficient relates to such propensity and thus to the amplitudes of the movements of the wearer's eyes and head.

A reference measurement of COT is carried out using a suitable device configured to assess the reference eye-head coefficient of the person.

Training

The training then consists in proposing visual targets or stimuli with different eccentricities, in the horizontal and vertical meridians but also randomly in the field of vision of the person. The person must detect the visual targets, discriminate and/or recognize them.

For example, the person must determine the orientation of Gabor patches displayed in the field of view of the person. Several parameters can further be modulated in order to make the task for the person more complex and thus to integrate the new eye-head behavior independently of the difficulty of the task. Thus, the contrast, the phase, the spatial frequency and/or the size of the Gabor patches can vary during training. Furthermore, the time sequencing can also be changed.

During the presentation of the visual stimuli, the rotation of the head is measured in real time using a tracking system, for example by inertial sensors, optical sensors and/or ultrasonic sensors.

During the training, a sensory feedback is associated with the recording of the movement of the head in order to signal to the person when it has carried out the adequate movement of the head. This feedback can be organized, for example, as follows:

- a beep sound accompanies the center target lights;
- then, a peripheral stimulus is then presented. If the person performs the requested task, for example determining the orientation of the Gabor patch, by mobilizing his head and eyes towards the target, a sound beep will indicate the quality of the task.

The evolution of the difficulties between the training sessions concerns the temporal sequencing, the uncertainty about the position of the targets, the accuracy of head rotation. The evolution of the difficulty between the sessions will be modulated according to a coefficient of performance CP obtained by the person, which may for example be calculated as follows:

$$CP = \frac{\sum (COT_D - COT_M)^2}{n}$$

Wherein:

- $COT_D$ the desired eye-head coordination/coefficient,
- $COT_M$ the measured eye-head coordination/coefficient, and
- n the number of targets proposed during the session.

Final person visual parameter assessment:

This final assessment aims to inform about the visual and/or visual-motor behaviour of the person after training and to allow to evaluate the effectiveness of the training. The final assessment is performed following the same protocol as the reference measurement.

**Example 3: Sensitivity to distortion**

Reference person visual parameter assessment:

This reference assessment aims to provide information on the reference visual and/or visual-motor behaviour of the person and to make it possible to judge the effectiveness of the adaptation by training. For example, the reference person visual parameter assessed is the sensitivity to distortions of the person.

A method to assess the sensitivity of a user to distortion is in particular disclosed in WO 2016/055 265.

A reference measurement of sensitivity is carried out using a viewing device of immersion type, virtual reality head mounted device... For the measurement, the person is positioned in a simulated visual environment in which distortions can be added in a controlled manner. Then, the reference measurement is to determine the effects of distortion on the visual and/or visuo-motor behavior of the person, such as object grasping, balance, walking, obstacle negotiation, eye-head coordination, in particular to position the gaze in the near vision area of a progressive power lens, etc.

This reference measure can also comprise determining the subjective sensitivity of the person to distortions. In this case, the person's visual judgment is required to measure whether or not he perceives the distortions.

Training

During the training, the person is exposed to increasing levels of distortion in order to assess the effects of these distortions and to make the person aware of how these distortions affect performance, behaviour, comfort, etc.

Then, a training is proposed to the person as described earlier, for instance on the basis of the measurements of the person relative to increasing levels of distortion.

**Example 4: Visual behaviour during sport**

Reference person visual parameter assessment:

This reference assessment aims to provide information on the reference visual and/or visual-motor behaviour of the sportsman/woman while wearing specific sport spectacle and to make it possible to judge the effectiveness of the adaptation by training. For example, the reference person visual parameter assessed is the eye movements pattern (EMp) specific to sport practice. While wearing wrap spectacle, the sportsman/woman may encounter difficulties to perform eccentric eye movements to gaze towards a ball and to intercept it with a tennis racket.

A reference measurement of EMp is carried out using a suitable device configured to assess the reference EMp of the sportsman/woman.

Training

During training, the person is exposed to different levels and eccentricities in order to assess the effect of these distortions and to make the sportsman aware of how these distortions affect his performance. The sportsman must follow one or more defined stimuli or visual targets among others, and/or react to avoid them or intercept them.

During the presentation of the visual stimuli, the eye movement pattern is measured in real time using a tracking system, for example by an eye tracker. Virtual reality systems such as a head mounted display can be used to measure the reaction of the person. The performance of the sportsman is measured in real time using a tracking system such as an active sensor. The goal is to assess the reaction of the sportsman looking at the stimuli.

During the training, a sensory feedback is associated with the recording of the Eye Movement pattern in order to signal to the person when he has carried out the adequate pattern. For example, an adequate EM pattern is the eccentricity of eye rotations done related to the distorted zone in the lens. A threshold of maximal eye movement eccentricity is defined to avoid using of distorted area and to avoid a decreasing performance; in this case, the optical parameter is the distortion of the lens.

This feedback can be organized, for example, as follows:

- a visualization of the amount of the eye rotation related to the threshold;
- a feedback on said amount;
- a feedback on the performance of sport wearer;
- then, if the sportsman performs poorly the task, i.e. if the amount of the eye movement is greater than the desired threshold, a similar presentation of visual targets or stimuli is displayed with an equivalent or lower task difficulty. If the person performs the requested task, a sound will indicate him the achievement of the task.

Final person visual parameter assessement:

This final assessment aims to inform about the visual and/or visual-motor behaviour of the person after training and to allow to evaluate the effectiveness of the training. The final assessment is performed following the same protocol as the reference measurement.

## Example 5: Perception of the curvature shapes

Any ophthalmic lens induces distortions, which can be simply defined, for this example, as a straight line that is seen as curved when the light rays travel through the lens. A particular case of visual distortion is present when progressive lenses are used because the distortions are asymmetrical with generally more curvature generated in the lower visual field induced by the continuous change of power in the lower part of the lens.

Reference person visual parameter assessment:

This reference assessment aims to provide information on the reference perception of curvature shapes of the person while wearing progressive lenses and to make it possible to judge the effectiveness of the adaptation to the distortions induced by the progressive lenses by training.

Training

During training, the person is exposed to different stimuli having distortions of different levels in order to assess the effect of these distortions and to make the person aware of how these distortions affect his perception of curvatures.

A training could for instance consist in presenting different stimuli to the user equipped with the ophthalmic lenses and in asking him to distinguish between real distortions and distortions induced by the ophthalmic lenses.

Final person visual parameter assessement:

This final assessment aims to inform about the perception of curvatures shapes of the person after training and to allow the evaluation of the effectiveness of the training. The final assessment is performed following the same protocol as the reference measurement.

Advantageously, the training of the person to perceive and understand the changes in curvatures induced by the progression of power allows the person to then more easily distinguish the curvatures and the natural shapes of the objects from those caused by the lens.

## Other examples

Other person visual parameter can be assessed.

For example, during a first wear/usage of (real or virtual) lens, the main problem encountered by the person can be determined among blurring, distortion, accessibility to the areas of the lens, eye-head coordination... The determination of the main problem can be carried out by means of a questionnaire, for example subjectively measuring the level of annoyance felt for each of the problems, or by objective measurements, such as the impact of optical constraints (blurring, distortion, accessibility to lens areas, restriction of the field of vision) on the visual, visuomotor or motor performance of the person.

According to another example, the adaptability of the person for each of the problems (blurring, distortion, accessibility to the glass, eye-head coordination/-coefficient) can be estimated to propose an optimized lens, the lens being optimized on the problem or problems for which the person has only a few of adaptability, and propose a training for the problem or problems for which the person has a good capacity of adaptation.

The method and the device according to the invention is particularly well-suitable for presbyopic persons but also for myopic children as well as for persons with high anisometropia between the two eyes.

Indeed, the invention is intended for the wearers or the future wearers of multifocal lenses. It makes it possible to improve the speed of adaptation to multifocal lenses for new wearers, to adapt to the wearing of multifocal lenses in the case of wearers experiencing difficulties in wearing multifocal lenses and to facilitate the transition between two multifocal lenses, for example in the case of a passage from bifocals to progressive lenses. From a visual perception point of view, a training will be aimed at a preference for low spatial frequencies to the detriment of high spatial frequencies.

The invention is also intended for children wearing peripheral correction lenses. These lenses inducing aberrations anywhere outside the optical axis, the visualmotor training will consist in making them as head-mover as possible. The perceptual training will aim to reduce the sensitivity to the blur to be less generated during ocular movements outside the optical axis of the lens.

Furthermore, numerous studies have shown that wearing of progressive or bifocal lenses can slow down the progression of myopia of children. However, the effectiveness of these lenses depends on the use of the addition area for near vision. As a child is not presbyopic, he/she does not need to use this area of the lens and can content himself/herself with lowering his/her head only to see up near. In this case, the training aims to make the child more head-mover vertically, in order to force him/her to use the area of near vision of the progressive or bifocal lens. The expected result is a better control of the progression of myopia.

Moreover, recent studies have shown that accommodative imprecision or accommodative lag seems to be involved in myopic progression of children. In addition, it was shown that the accommodative lag was greater in myopic children than in emmetropic children. It has also been shown that myopes are less sensitive to blur, which may in part explain the presence of larger accommodative lags. In the case of myopic children, the pethood allows improving the accuracy of accommodation (reduce lag) in order to reduce myopic progression in children. An inaccurate accommodation (lag) has a low pass filter effect on the spatial frequencies of the image. Consequently, it would be useful to favor the preference for fine details, ie high spatial frequencies. The training according to the invention will therefore involve only training on visual perception and will aim at a preference for high spatial frequencies at the expense of low spatial frequencies.

Furthermore, the invention can also be intended for anisometropic ametropes. Indeed, the correction in spectacle lenses of an anisometropia creates differences in magnification and prismatic effects off the optical axis which can be extremely great, especially in near vision for the presbyopic anisometrope person. Consequently, the method according to the invention comprises advantageously a training step for training the anisometropes in a monocular manner in order to prefer the low spatial frequencies of an eye (preferably the most ametropic eye) in order to be able to under-correct the latter in order to reduce the difference in magnification and prism. In another embodiment, the training can also cause the opposite eye to prefer high spatial frequencies.

The invention has been described above with the aid of embodiments without limitation of the general inventive concept, as defined in the enclosed set of claims.

Many further modifications and variations will suggest themselves to those skilled in the art upon making reference to the foregoing illustrative embodiments, which are given by way of example only and which are not intended to limit the scope of the invention, that being determined solely by the appended claims.

In the claims, the word "comprising" does not exclude other elements or steps, and the indefinite article "a" or "an" does not exclude a plurality. The mere fact that different features are recited in mutually different dependent claims does not indicate that a combination of these features cannot be advantageously used. Any reference signs in the claims should not be construed as limiting the scope of the invention.

## Claims

1. A computer-implemented method for adapting the visual and/or visual-motor behaviour of a person, the method comprising:

- **an optical parameter providing step (S2),** during which an optical parameter characterizing an optical system is provided, the optical system being intended to equip the person;
- **a person visual parameter providing step (S4),** during which a person visual parameter indicative of a visual parameter of the person relative to a given visual and/or visual-motor behaviour of the person is provided;
- **a target value providing step (S6),** during which a target value for the person visual parameter related to the optical parameter is provided;
- **a visual and/or visual motor behaviour modifying step (S8),** during which a reference visual and/or visual-motor behaviour of the person is modified so as to define a modified visual and/or visual-motor behaviour of the person by providing a visual and/or visual-motor training to the person;
- **a person visual parameter assessing step (S10),** during which the person visual parameter is assessed for the modified visual and/or visual-motor behaviour of the person;
- **a determining step (S12),** during which a suitable visual and/or visual-motor behaviour for the person equipped with the optical system is determined by repeating the modifying and assessing steps so as to minimize the difference between the assessed person visual parameters and the target value; and
- **an adapting step (S14),** during which the visual and/or visual-motor behaviour of the person equipped with the optical system is adapted based on the suitable visual and/or visual-motor behaviour.

wherein the visual parameter of the person relative to a given visual and/or visual-motor behaviour of the person comprises:

- a visual behaviour parameter and/or,
- a visual-motor parameter and/or,
- a visual performance parameter and/or,
- a curvature visual perception data,

wherein the visual-motor parameter is indicative of the visual-motor behaviour of the person, preferably visual-motor coordination data representative of the visual-motor coordination of the person.

2. Method according to claim 1, wherein the person visual parameter comprises:

- a visual behaviour parameter indicative of the visual behaviour of the person; and/or
- a visual sensitivity parameter, indicative of the visual sensitivity of the person, preferably optical distortion sensitivity data representative of the sensitivity of the person to optical distortions and/or blur tolerance data representative of the tolerance of the person to blur; and/or
- a visual performance parameter indicative of the visual performance of the person; and/or
- a curvature visual perception data representative of the visual perception of the person for curvature shapes.

3. Method according to claim 1 or 2, wherein the optical system comprises at least an ophthalmic lens, preferably a progressive addition lens.

4. Method according to claim 3, wherein the optical parameter of the ophthalmic lens comprises at least lens design data indicative of a lens design adapted to the person, the lens design comprising at least the dioptric lens design and/or geometrical parameters of the ophthalmic lens and/or prescription data and/or ophthalmic parameters relating to the ophthalmic requirements of the person.

5. The method according to any of the preceding claims 1 to 4, wherein the person is equipped with the optical system during the visual and/or visual-motor behaviour modifying step.

6. The method according to any of the preceding claims 1 to 5, further comprising a person visual parameter measurement step (S16), during which the person visual parameter of the person relative to at least one visual and/or visual-motor behaviour of the person is measured.

7. The method according to claim 6, wherein the measured value of the person visual parameter is compared to a scale to define a person visual index.

8. The method according to claim 6 or 7, wherein the person visual parameter is measured upon a perception test carried out on the person.

9. The method according to any one of the claims 6 to 8, further comprising a person visual parameter flexibility measuring step (S18), during which the flexibility of the person visual parameter is measured based on the measurement of the person visual parameter.

10. The method according to any of the preceding claims 1 to 9, wherein the visual and/or visual-motor behaviour modifying step comprises at least:

- a visual pattern presentation step, during which a set of visual patterns is presented to the person, the set of visual patterns having a visual pattern parameter whose value for each visual pattern varies in the set, the visual pattern parameter being related to the person visual parameter;
- a reference value providing step, during which a reference value of the visual parameter of the person based on said visual patterns and on said optical parameter of the optical system is provided,
- a comparing step, during which the person visual parameter and the reference value are compared so as to deduce whether the use of the optical system by the person is adapted when the person is equipped with the optical system and when the visual patterns are looked by the person, and
- a warning step, during which a warning is provided to the person based on the result of the comparing step.

11. The method according to any of the preceding claims 1 to 10, further comprising a visual and/or visual-motor behaviour adapting efficiency evaluating step (S20), during which the efficiency of the visual and/or visual-motor behaviour adapting is evaluated.

12. The method according to claim 11, further comprising an optical system information generating step (S22), during which an information related to an optical system adapted to the person is generated based on the efficiency of the visual and/or visual-motor behaviour adapting.

13. A computer program product comprising one or more stored sequences of instructions that are accessible to a processor and which, when executed by the processor, causes the processor to carry out the steps of any of claims 1 to 12.

14. A system for adapting the visual and/or visual-motor behaviour of a person, the system comprising:

- first receiving means (12) adapted to receive an optical parameter of an optical system, the optical system being intended to equip the person,
- second receiving means (14) adapted to receive a person visual parameter indicative of a visual parameter of the person relative to a given visual and/or visual-motor behaviour of the person,
- third receiving means (16) adapted to receive a target value for the person visual parameter related to the optical parameter;
- visual and/or visual-motor behaviour modifying means (18) adapted to modify a reference visual and/or visual-motor behaviour of the person so as to define a modified visual and/or visual-motor behaviour of the person by providing a visual and/or visual-motor training to the person;
- visual parameter assessing means (20) adapted to assess the person visual parameter for the modified visual and/or visual-motor behaviour of the person;
- determining means (22) adapted to determine a suitable visual and/or visual-motor behaviour for the person equipped with the optical system among the modified visual and/or visual-motor behaviours, the visual and/or visual-motor behaviour modifying means (18) and the visual parameter assessing means (20) being configured to repeat the modification of the reference visual and/or visual-motor behaviour of the person and the assessment of his/her corresponding person visual parameter so as to minimize the difference between the assessed person visual parameters and the target value; and
- adapting means (24) configured to adapt the visual and/or visual-motor behaviour of the person equipped with the optical system based on the suitable visual and/or visual-motor behaviour.

wherein the visual parameter of the person relative to a given visual and/or visual-motor behaviour of the person comprises:

- a visual behaviour parameter and/or,
- a visual-motor parameter and/or,
- a visual performance parameter and/or,
- a curvature visual perception data,

wherein the visual-motor parameter is indicative of the visual-motor behaviour of the person, preferably visual-motor coordination data representative of the visual-motor coordination of the person.

15. A system according to claim 14, wherein the visual and/or visual-motor behaviour adapting means (24) comprise at least a mobile device, for example a smartphone and the second receiving means (14) comprise a camera embedded in the mobile device configured to receive the person visual parameter.

**Patentansprüche**

1. Computerimplementiertes Verfahren zum Anpassen des visuellen und/oder visuell-motorischen Verhaltens einer Person, wobei das Verfahren Folgendes umfasst:

- einen Schritt (S2) des Bereitstellens eines optischen Parameters, bei dem ein optischer Parameter, der ein optisches System charakterisiert, bereitgestellt wird, wobei das optische System vorgesehen ist, um die Person auszustatten;
- einen Schritt (S4) des Bereitstellens eines visuellen Parameters für eine Person, bei dem ein visueller Parameter für eine Person bereitgestellt wird, der einen visuellen Parameter der Person relativ zu einem gegebenen visuellen und/oder visuell-motorischen Verhalten der Person angibt;
- einen Schritt (S6) des Bereitstellens eines Zielwerts, bei dem ein Zielwert für den visuellen Parameter der Person bereitgestellt wird, der in Beziehung zu dem optischen Parameter steht;
- einen Schritt (S8) des Modifizierens des visuellen und/oder visuell-motorischen Verhaltens, bei dem ein visuelles und/oder visuell-motorisches Referenzverhalten der Person so modifiziert wird, dass ein modifiziertes visuelles und/oder visuell-motorisches Verhalten der Person definiert wird, indem der Person ein visuelles und/oder visuell-motorisches Training bereitgestellt wird;
- einen Schritt (S10) des Beurteilens der visuellen Parameter der Person, bei dem der visuelle Parameter der Person für das modifizierte visuelle und/oder visuell-motorische Verhalten der Person beurteilt wird;
- einen Bestimmungsschritt (S12), bei dem ein geeignetes visuelles und/oder visuell-motorisches Verhalten für die Person, die mit dem optischen System ausgestattet ist, bestimmt wird, indem die Modifizierungs- und Beurteilungsschritte wiederholt werden, um die Differenz zwischen den visuellen Parametern der beurteilten Person und dem Zielwert zu minimieren; und
- einen Anpassungsschritt (S14), bei dem das visuelle und/oder visuell-motorische Verhalten der Person, die mit dem optischen System ausgestattet ist, basierend auf dem geeigneten visuellen und/oder visuell-motorischen Verhaltens angepasst wird,

wobei der visuelle Parameter der Person relativ zu einem gegebenen visuellen und/oder visuell-motorischen Verhalten der Person umfasst:

- einen visuellen Verhaltensparameter und/oder
- einen visuell-motorischen Parameter und/oder
- einen visuellen Leistungsfähigkeitsparameter und/oder
- Daten der gekrümmten visuellen Wahrnehmung,

wobei der visuell-motorische Parameter das visuell-motorische Verhalten der Person angibt, vorzugsweise visuell-motorische Koordinationsdaten, die für die visuell-motorische Koordination der Person repräsentativ sind.

2. Verfahren nach Anspruch 1, wobei der visuelle Parameter der Person Folgendes umfasst:

- einen visuellen Verhaltensparameter, der das visuelle Verhalten der Person angibt; und/oder
- einen visuellen Empfindlichkeitsparameter, der die visuelle Empfindlichkeit der Person angibt, vorzugsweise optische Verzerrungsempfindlichkeitsdaten, die die Empfindlichkeit der Person gegenüber optischen Verzerrungen repräsentieren, und/oder Unschärfetoleranzdaten, die die Toleranz der Person gegenüber Unschärfe repräsentieren; und/oder
- einen visuellen Leistungsfähigkeitsparameter, der die visuelle Leistungsfähigkeit der Person angibt; und/oder
- Daten der gekrümmten visuellen Wahrnehmung, die die visuelle Wahrnehmung der Person für Krümmungsformen repräsentieren.

3. Verfahren nach Anspruch 1 oder 2, wobei das optische System mindestens eine ophthalmische Linse, vorzugsweise eine progressive Additionslinse, umfasst.

4. Verfahren nach Anspruch 3, wobei der optische Parameter der ophthalmischen Linse mindestens Linsendesigndaten umfasst, die ein an die Person angepasstes Linsendesign angeben, wobei das Linsendesign mindestens das dioptrische Linsendesign und/oder geometrische Parameter der ophthalmischen Linse und/oder Verschreibungsdaten und/oder ophthalmische Parameter in Bezug auf die ophthalmischen Anforderungen der Person umfasst.

5. Verfahren nach einem der vorhergehenden Ansprüche 1 bis 4, wobei die Person mit dem optischen System während des Schrittes des Modifizierens des visuellen und/oder visuell-motorischen Verhaltens ausgestattet wird.

6. Verfahren nach einem der vorhergehenden Ansprüche 1 bis 5, ferner umfassend einen Schritt (S16) zum Messen visueller Parameter der Person, bei dem der visuelle Parameter der Person relativ zu mindestens einem visuellen und/oder visuell-motorischen Verhalten der Person gemessen wird.

7. Verfahren nach Anspruch 6, wobei der gemessene Wert des visuellen Parameters der Person mit einer Skala verglichen wird, um einen visuellen Index der Person zu definieren.

8. Verfahren nach Anspruch 6 oder 7, wobei der visuelle Parameter der Person bei einem Wahrnehmungstest gemessen wird, der an der Person durchgeführt wird.

9. Verfahren nach einem der Ansprüche 6 bis 8, ferner umfassend einen Schritt (S18) zum Messen der Flexibilität visueller Parameter für eine Person, bei dem die Flexibilität des visuellen Parameters für eine Person basierend auf der Messung des visuellen Parameters für eine Person gemessen wird.

10. Verfahren nach einem der vorhergehenden Ansprüche 1 bis 9, wobei der Schritt des Modifizierens des visuellen und/oder visuell-motorischen Verhaltens mindestens Folgendes umfasst:

- einen Schritt des Präsentierens visueller Muster, bei dem der Person ein Satz visueller Muster präsentiert wird, wobei der Satz visueller Muster einen visuellen Musterparameter aufweist, dessen Wert für jedes visuelle Muster in dem Satz variiert, wobei der visuelle Musterparameter mit dem visuellen Parameter der Person in Beziehung steht;
- einen Schritt des Bereitstellens eines Referenzwerts, bei dem ein Referenzwert des visuellen Parameters der Person basierend auf den visuellen Mustern und auf dem optischen Parameter des optischen Systems bereitgestellt wird,
- einen Vergleichsschritt, bei dem der visuelle Parameter der Person und der Referenzwert verglichen werden, um daraus zu schließen, ob die Verwendung des optischen Systems durch die Person angepasst ist, wenn die Person mit dem optischen System ausgestattet ist und wenn die visuellen Muster von der Person betrachtet werden, und
- einen Warnschritt, bei dem der Person basierend auf dem Ergebnis des Vergleichsschritts eine Warnung bereitgestellt wird.

11. Verfahren nach einem der vorhergehenden Ansprüche 1 bis 10, ferner umfassend einen Schritt (S20) des Bewertens der Anpassungseffizienz des visuellen und/oder visuell-motorischen Verhaltens, bei dem die Effizienz der Anpassung des visuellen und/oder visuell-motorischen Verhaltens bewertet wird.

12. Verfahren nach Anspruch 11, ferner umfassend einen Schritt (S22) des Generierens von Informationen des optischen Systems, bei dem Informationen, die mit einem optischen System in Beziehung stehen, das an die Person angepasst ist, basierend auf der Anpassungseffizienz des visuellen und/oder visuell-motorischen Verhaltens generiert werden.

---

**13.** Computerprogrammprodukt, umfassend eine oder mehrere gespeicherte Sequenzen von Anweisungen, auf die ein Prozessor zugreifen kann und die, wenn sie von dem Prozessor ausgeführt werden, den Prozessor zum Ausführen der Schritte nach einem der Ansprüche 1 bis 12 veranlassen.

**14.** System zum Anpassen des visuellen und/oder visuell-motorischen Verhaltens einer Person, wobei das System Folgendes umfasst:

- erste Empfangsmittel (12), die dazu angepasst sind, einen optischen Parameter eines optischen Systems zu empfangen, wobei das optische System vorgesehen ist, die Person auszustatten,
- zweite Empfangsmittel (14), die dazu angepasst sind, einen visuellen Parameter der Person zu empfangen, der einen visuellen Parameter der Person relativ zu einem gegebenen visuellen und/oder visuell-motorischen Verhalten der Person angibt,
- dritte Empfangsmittel (16), die dazu angepasst sind, einen Zielwert für den visuellen Parameter der Person zu empfangen, der in Beziehung zu dem optischen Parameter steht;
- Mittel (18) zum Modifizieren des visuellen und/oder visuell-motorischen Verhaltens, die dazu angepasst sind, ein visuelles und/oder visuell-motorisches Referenzverhalten der Person zu modifizieren, um ein modifiziertes visuelles und/oder visuell-motorisches Verhalten der Person zu definieren, indem der Person ein visuelles und/oder visuell-motorisches Training bereitgestellt wird;
- Mittel (20) zum Beurteilen visueller Parameter, die dazu angepasst sind, den visuellen Parameter der Person für das modifizierte visuelle und/oder visuell-motorische Verhalten der Person zu beurteilen;
- Bestimmungsmittel (22), die dazu angepasst sind, ein geeignetes visuelles und/oder visuell-motorisches Verhalten für die Person, die mit dem optischen System ausgestattet ist, innerhalb der modifizierten visuellen und/oder visuell-motorischen Verhaltensweisen zu bestimmen, wobei die Mittel (18) zum Modifizieren des visuellen und/oder visuell-motorischen Verhaltens und die Mittel (20) zum Beurteilen des visuellen Parameters dazu ausgelegt sind, die Modifikation des visuellen und/oder visuell-motorischen Referenzverhaltens der Person und die Beurteilung ihres entsprechenden visuellen Parameters zu wiederholen, um die Differenz zwischen den visuellen Parametern der beurteilten Person und dem Zielwert zu minimieren; und
- Anpassungsmittel (24), die dazu ausgelegt sind, das visuelle und/oder visuell-motorische Verhalten der Person, die mit dem optischen System ausgestattet ist, basierend auf dem geeigneten visuellen und/oder visuell-motorischen Verhalten anzupassen;
wobei der visuelle Parameter der Person relativ zu einem gegebenen visuellen und/oder visuell-motorischen Verhalten der Person umfasst:

- einen visuellen Verhaltensparameter und/oder
- einen visuell-motorischen Parameter und/oder
- einen visuellen Leistungsfähigkeitsparameter und/oder
- Daten der gekrümmten visuellen Wahrnehmung,

wobei der visuell-motorische Parameter das visuell-motorische Verhalten der Person angibt, vorzugsweise visuell-motorische Koordinationsdaten, die für die visuell-motorische Koordination der Person repräsentativ sind.

**15.** System nach Anspruch 14, wobei die Mittel (24) zum Anpassen des visuellen und/oder visuell-motorischen Verhaltens mindestens eine mobile Vorrichtung, zum Beispiel ein Smartphone, umfassen, und das zweite Empfangsmittel (14) eine Kamera umfasst, die in die mobile Vorrichtung eingebettet ist und dazu ausgelegt ist, den visuellen Parameter der Person zu empfangen.

## Revendications

**1.** Procédé mis en œuvre par ordinateur pour adapter le comportement visuel et/ou visuomoteur d'une personne, le procédé comprenant :

- une étape d'obtention de paramètre optique (S2), lors de laquelle un paramètre optique caractérisant un système optique est obtenu, le système optique étant destiné à équiper la personne ;
- une étape d'obtention de paramètre visuel personnel (S4), lors de laquelle un paramètre visuel personnel indiquant un paramètre visuel personnel relatif à un comportement visuel et/ou visuomoteur donné de la personne est obtenu ;
- une étape d'obtention de valeur cible (S6), lors de laquelle une valeur cible pour le paramètre visuel personnel lié au paramètre optique est obtenue ;
- une étape de modification de comportement visuel et/ou visuomoteur (S8), lors de laquelle un comportement visuel et/ou visuomoteur de référence de la personne est modifié de manière

14

à définir un comportement visuel et/ou visuomoteur modifié de la personne par fourniture d'un entraînement visuel et/ou visuomoteur à la personne ;

- une étape d'évaluation de paramètre visuel personnel (S10), lors de laquelle le paramètre visuel personnel est évalué pour le comportement visuel et/ou visuomoteur modifié de la personne ;

- une étape de détermination (S12), lors de laquelle un comportement visuel et/ou visuomoteur approprié pour la personne équipée du système optique est déterminé par répétition des étapes de modification et d'évaluation de manière à minimiser la différence entre les paramètres visuels personnels évalués et la valeur cible ; et

- une étape d'adaptation (S14), lors de laquelle le comportement visuel et/ou visuomoteur de la personne équipée du système optique est adapté en fonction du comportement visuel et/ou visuomoteur approprié,

dans lequel le paramètre visuel de la personne relatif à un comportement visuel et/ou visuomoteur donné de la personne comprend :

- un paramètre de comportement visuel, et/ou
- un paramètre visuomoteur, et/ou
- un paramètre de performance visuelle, et/ou
- une donnée de perception visuelle des courbes,

dans lequel le paramètre visuomoteur indique le comportement visuomoteur de la personne, de préférence des données de coordination visuomotrice représentatives de la coordination visuomotrice de la personne.

**2.** Procédé selon la revendication 1, dans lequel le paramètre visuel personnel comprend :

- un paramètre de comportement visuel indiquant le comportement visuel de la personne ; et/ou
- un paramètre de sensibilité visuelle, indiquant la sensibilité visuelle de la personne, de préférence des données de sensibilité à la distorsion optique représentatives de la sensibilité de la personne aux distorsions optiques et/ou des données de tolérance au flou représentatives de la tolérance de la personne au flou ; et/ou
- un paramètre de performance visuelle indiquant la performance visuelle de la personne ; et/ou
- une donnée de perception visuelle des courbes représentative de la perception visuelle de la personne pour des formes courbes.

**3.** Procédé selon la revendication 1 ou 2, dans lequel le système optique comprend au moins une lentille ophtalmique, de préférence une lentille progressive.

**4.** Procédé selon la revendication 3, dans lequel le paramètre optique de la lentille ophtalmique comprend au moins des données de conception de lentille indiquant une conception de lentille adaptée à la personne, la conception de lentille comprenant au moins la conception de lentille dioptrique et/ou des paramètres géométriques de la lentille ophtalmique et/ou des données de prescription et/ou des paramètres ophtalmiques relatifs aux exigences ophtalmiques de la personne.

**5.** Procédé selon l'une quelconque des revendications 1 à 4 précédentes, dans lequel la personne est équipée du système optique lors de l'étape de modification de comportement visuel et/ou visuomoteur.

**6.** Procédé selon l'une quelconque des revendications 1 à 5 précédentes, comprenant en outre une étape de mesure de paramètre visuel personnel (S16), lors de laquelle le paramètre visuel personnel de la personne relatif à au moins un comportement visuel et/ou visuomoteur de la personne est mesuré.

**7.** Procédé selon la revendication 6, dans lequel la valeur mesurée du paramètre visuel personnel est comparée à une échelle pour définir un indice visuel personnel.

**8.** Procédé selon la revendication 6 ou 7, dans lequel le paramètre visuel personnel est mesuré lors d'un test de perception réalisé sur la personne.

**9.** Procédé selon l'une quelconque des revendications 6 à 8, comprenant en outre une étape de mesure de flexibilité de paramètre visuel personnel (S18), lors de laquelle la flexibilité du paramètre visuel personnel est mesurée en fonction de la mesure du paramètre visuel personnel.

**10.** Procédé selon l'une quelconque des revendications 1 à 9 précédentes, dans lequel l'étape de modification de comportement visuel et/ou visuomoteur comprend au moins :

- une étape de présentation de motifs visuels, lors de laquelle un ensemble de motifs visuels est présenté à la personne, l'ensemble de motifs visuels ayant un paramètre de motif visuel dont la valeur pour chaque motif visuel varie dans l'ensemble, le paramètre de motif visuel étant lié au paramètre visuel personnel ;

- une étape d'obtention de valeur de référence, lors de laquelle une valeur de référence du paramètre visuel de la personne basée sur lesdits motifs visuels et sur ledit paramètre optique du système optique est obtenue ;
- une étape de comparaison, lors de laquelle le paramètre visuel personnel et la valeur de référence sont comparés afin de déduire si l'utilisation du système optique par la personne est adaptée lorsque la personne est équipée du système optique et lorsque les motifs visuels sont regardés par la personne ; et
- une étape d'avertissement, lors de laquelle un avertissement est fourni à la personne en fonction du résultat de l'étape de comparaison.

11. Procédé selon l'une quelconque des revendications 1 à 10 précédentes, comprenant en outre une étape d'évaluation d'efficacité d'adaptation de comportement visuel et/ou visuomoteur (S20), lors de laquelle l'efficacité de l'adaptation du comportement visuel et/ou visuomoteur est évaluée.

12. Procédé selon la revendication 11, comprenant en outre une étape de génération d'information de système optique (S22), lors de laquelle une information liée à un système optique adapté à la personne est générée en fonction de l'efficacité de l'adaptation du comportement visuel et/ou visuomoteur.

13. Produit-programme informatique comprenant une ou plusieurs séquences d'instructions stockées qui sont accessibles à un processeur et qui, lors de leur exécution par le processeur, amènent le processeur à réaliser les étapes de l'une quelconque des revendications 1 à 12.

14. Système destiné à adapter le comportement visuel et/ou visuomoteur d'une personne, le système comprenant :

- de premiers moyens de réception (12) adaptés pour recevoir un paramètre optique d'un système optique, le système optique étant destiné à équiper la personne,
- de deuxièmes moyens de réception (14) adaptés pour recevoir un paramètre visuel personnel indiquant un paramètre visuel de la personne relatif à un comportement visuel et/ou visuomoteur donné de la personne,
- de troisièmes moyens de réception (16) adaptés pour recevoir une valeur cible pour le paramètre visuel personnel lié au paramètre optique,
- des moyens de modification de comportement visuel et/ou visuomoteur (18) adaptés pour modifier un comportement visuel et/ou visuomoteur de référence de la personne de manière à définir un comportement visuel et/ou visuomoteur modifié de la personne en fournissant un entraînement visuel et/ou visuomoteur à la personne,
- des moyens d'évaluation de paramètre visuel (20) adaptés pour évaluer le paramètre visuel personnel pour le comportement visuel et/ou visuomoteur modifié de la personne,
- des moyens de détermination (22) adaptés pour déterminer un comportement visuel et/ou visuomoteur approprié pour la personne équipée du système optique parmi les comportements visuels et/ou visuomoteurs modifiés, les moyens de modification de comportement visuel et/ou visuomoteur (18) et les moyens d'évaluation de paramètre visuel (20) étant conçus pour répéter la modification du comportement visuel et/ou visuomoteur de référence de la personne et l'évaluation de son paramètre visuel personnel correspondant de manière à minimiser la différence entre les paramètres visuels personnels évalués et la valeur cible, et
- des moyens d'adaptation (24) conçus pour adapter le comportement visuel et/ou visuomoteur de la personne équipée du système optique en fonction du comportement visuel et/ou visuomoteur approprié,
dans lequel le paramètre visuel de la personne relatif à un comportement visuel et/ou visuomoteur donné de la personne comprend :

- un paramètre de comportement visuel, et/ou
- un paramètre visuomoteur, et/ou
- un paramètre de performance visuelle, et/ou
- une donnée de perception visuelle des courbes,

dans lequel le paramètre visuomoteur indique le comportement visuomoteur de la personne, de préférence des données de coordination visuomotrice représentatives de la coordination visuomotrice de la personne.

15. Système selon la revendication 14, dans lequel les moyens d'adaptation de comportement visuel et/ou visuomoteur (24) comprennent au moins un dispositif mobile, par exemple un mobile multifonction, et les deuxièmes moyens de réception (14) comprennent une caméra intégrée dans le dispositif mobile conçue pour recevoir le paramètre visuel personnel.

**Figure 1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015124574 A1
- WO 2017144933 A1
- JP 2003075783 A
- US 20170017083 A1
- US 20100002191 A
- WO 2016055265 A

**Non-patent literature cited in the description**

- **S. GONG et al.** *Image and Vision Computing*, 2002, vol. 20, 873-888
- **O. ELOKA et al.** *Vision Research*, 2011, vol. 51, 925-931